# EUROPÄISCHE PATENTANMELDUNG

(11) **EP 4 603 627 A1**
(43) Veröffentlichungstag der Anmeldung: **20.08.2025**
(21) Anmeldenummer: 25156750.9
(22) Anmeldetag: 10.02.2025
(51) Int. Cl.: D01H 13/22, G01B 11/10, G01N 21/89, G01N 33/36

(54) **VERFAHREN ZUR ERKENNUNG EINER ENTSTEHUNG VON WENIGSTENS EINER SCHWACHSTELLE EINES GESPONNENEN GARNS, VORRICHTUNG ZUR DATENVERARBEITUNG, ÜBERWACHUNGSVORRICHTUNG, SPINNMASCHINE, COMPUTERPROGRAMM UND COMPUTERLESBARES MEDIUM**

(30) Priorität: 19.02.2024 LU 506413
(71) Anmelder: Saurer Spinning Solutions GmbH & Co. KG, 52531 Übach-Palenberg (DE)
(72) Erfinder: Radermacher, Wolfgang, 41844 Wegberg (DE); Hüls, Jürgen, 40670 Meerbusch (DE); Mohr, Hans-Peter, 41189 Mönchengladbach (DE)
(74) Vertreter: Morgenthum-Neurode, Mirko

(57) **Zusammenfassung**

Die Erfindung betrifft ein Verfahren zur Erkennung einer Entstehung von wenigstens einer Schwachstelle gesponnenen, insbesondere luftgesponnenen, Garns bei einer Spinnmaschine, aufweisend die nachfolgenden Schritte:
- Empfangen von mindestens einem Erfassungswert, welcher aus einer optischen Erfassung des Garns resultiert, wobei der mindestens eine Erfassungswert für eine Helligkeit des Garns spezifisch ist,
- Auswerten des mindestens einen Erfassungswerts, um eine Unregelmäßigkeit in Bezug auf die Helligkeit des Garns festzustellen,
- Erkennen der Entstehung der wenigstens einen Schwachstelle auf Basis der festgestellten Unregelmäßigkeit.

Ferner betrifft die Erfindung eine Vorrichtung zur Datenverarbeitung, Überwachungsvorrichtung (306), eine Spinnmaschine, ein Computerprogramm sowie
ein computerlesbares Medium.

## Beschreibung

Die vorliegende Erfindung betrifft ein Verfahren zur Erkennung einer Entstehung von wenigstens einer Schwachstelle eines gesponnenen, insbesondere luftgesponnenen, Garns, eine Vorrichtung zur Datenverarbeitung, eine Überwachungsvorrichtung, eine Spinnmaschine, ein Computerprogramm und ein computerlesbares Medium.

### Stand der Technik

Eine sichere Erkennung einer Entstehung von Schwachstellen eines gesponnenen, insbesondere luftgesponnenen, Garns ist häufig kaum oder sehr spät möglich. Derartige Schwachstellen sind ein bekanntes Problem im Besonderen bei luftgesponnenem Garn und werden sehr spät oder teilweise gar nicht erkannt. Kleinere und größere Längen mit fehlerhaftem Garnstellen gelangen somit auf die Spule, was bei der Weiterverarbeitung zu Problemen mit größeren Stillstandzeiten und Mehrkosten führen kann.

Die Druckschrift EP 2 169 097 A1 offenbart eine Vorrichtung zur Detektion von Fremdsubstanzen an gesponnenem Garn.

Die Druckschrift WO 2021/223784 A1 offenbart ein Verfahren zur Detektion von vollständig ausgebildeten Strukturfehlern gesponnenen Garns mittels eines Fotoabgleichs.

Insbesondere ist es eine Aufgabe der Erfindung, eine, insbesondere alternative oder verbesserte, Lösung zur Erkennung einer Entstehung von wenigstens einer Schwachstelle eines gesponnenen, insbesondere luftgesponnenen, Garns bereitzustellen, welche sich insbesondere durch ihre kostengünstige Ausführbarkeit und vorzugsweise durch ihre sichere und frühzeitige Erkennung der wenigstens einen Schwachstelle des gesponnen, insbesondere luftgesponnenen, Garns auszeichnet.

Die voranstehende Aufgabe wird gelöst durch ein Verfahren, eine Vorrichtung zur Datenverarbeitung, eine Überwachungsvorrichtung, eine Spinnmaschine, ein Computerprogramm sowie durch ein computerlesbares Medium mit den Merkmalen der entsprechenden unabhängigen Ansprüche. Weitere Merkmale und Details der Erfindung ergeben sich aus den jeweiligen Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit der erfindungsgemäßen Vorrichtung zur Datenverarbeitung, der erfindungsgemäßen Überwachungsvorrichtung, der erfindungsgemäßen Spinnmaschine, dem erfindungsgemäßen Computerprogramm und dem erfindungsgemäßen computerlesbaren Medium, und jeweils umgekehrt, sodass bezüglich der Offenbarung zu den einzelnen Erfindungsaspekten stets wechselseitig Bezug genommen wird bzw. werden kann.

### Offenbarung der Erfindung

Die Aufgabe wird nach einem Aspekt insbesondere durch ein Verfahren zur Erkennung einer Entstehung von wenigstens einer Schwachstelle eines gesponnenen, insbesondere luftgesponnenen, Garns bei einer Spinnmaschine gelöst.

Das erfindungsgemäße Verfahren umfasst gemäß einem ersten Verfahrensschritt das Empfangen von mindestens einem Erfassungswert, welcher aus einer optischen Erfassung des Garns resultiert, wobei der mindestens eine Erfassungswert für eine Helligkeit des Garns spezifisch ist, d. h., insbesondere für die von dem Garn abgestrahlte Lichtmenge, welche das Garn aufgrund einer Beleuchtung reflektiert (auch: remittiert), bevorzugt diffus reflektiert. Der Erfassungswert kann somit ein Helligkeitswert und/oder -grad sein, welcher vorzugsweise das Ausmaß der Remission angibt, die von dem Garn ausgeht. Um die optische Erfassung des Garns zu ermöglichen, kann daher eine Beleuchtung des Garns mit einer vordefinierten Lichtstärke und/oder Beleuchtungsstärke vorgesehen sein.

Das erfindungsgemäße Verfahren umfasst gemäß einem weiteren Verfahrensschritt das Auswerten des mindestens einen Erfassungswerts, um eine Unregelmäßigkeit in Bezug auf die Helligkeit des Garns festzustellen. Insbesondere kann hierbei eine Abweichung der von dem Garn abgestrahlten Lichtmenge zu wenigstens einer Referenz-Lichtmenge ermittelt werden, bevorzugt von einem Referenz-Lichtmengenbereich (Referenz). Die Auswertung ist z. B. mittels einer digitalen Verarbeitung des mindestens einen Erfassungswerts vorgesehen, z. B. durch einen numerischen Vergleich des mindestens einen Erfassungswerts oder mehrerer Erfassungswerte und der Referenz.

Ferner umfasst das erfindungsgemäße Verfahren gemäß einem weiteren Verfahrensschritt das Erkennen der Entstehung der wenigstens einen Schwachstelle auf Basis der festgestellten Unregelmäßigkeit. Hierzu ist es beispielsweise vorgesehen, dass die Abweichung überprüft wird und/oder bei dem Detektieren der Abweichung, insbesondere nach der Detektion über einen vordefinierten Zeitraum, ein Warnsignal ausgegeben wird. Hierdurch wird ein Verfahren zur Erkennung einer Entstehung von wenigstens einer Schwachstelle eines gesponnenen, insbesondere luftgesponnenen, Garns bereitgestellt, welches sich insbesondere durch seine kostengünstige Ausführbarkeit und vorzugsweise durch seine sichere und frühzeitige Erkennung der wenigstens einen Schwachstelle des gesponnenen, insbesondere luftgesponnenen, Garns auszeichnet. Dabei ist es bevorzugt, wenn das Empfangen zeitlich vor dem Auswerten und das Auswerten zeitlich vor dem Erkennen erfolgt.

Es ist ein besonderer Vorteil, der sich aus dem erfindungsgemäßen Verfahren ergeben kann, dass im Gegensatz zu herkömmlichen Lösungen nicht (nur) die ausgebildete Schwachstelle erkannt wird, sondern die Schwachstelle bereits in den frühen Phasen ihrer Entstehung erkannt werden kann, um frühzeitig Maßnahmen gegen die Ausbildung der Schwachstelle ergreifen zu können.

Es ist zweckmäßig, wenn das Verfahren während eines Spinnvorgangs, insbesondere während eines Luftspinnvorgangs, erfolgt, wobei mit dem Spinnvorgang Garn gesponnen, insbesondere luftgesponnen, wird, welches durch das erfindungsgemäße Verfahren auf eine Entstehung von wenigstens einer Schwachstelle überwacht werden soll. Der Spinnvorgang kann mittels einer Spinnmaschine, insbesondere mit einer Spinnstelle einer Spinnmaschine, durchgeführt werden. Bei der Spinnmaschine handelt es sich vorzugsweise um eine Luftspinnmaschine, welche eine Vielzahl von Spinnstellen aufweisen kann. Alternativ kann es sich bei der Spinnmaschine auch um eine Rotorspinnmaschine handeln. Die Verfahrensschritte können bevorzugt wiederholt ausgeführt werden, insbesondere während des Spinnvorgangs.

Grundsätzlich ist es bevorzugt, wenn an jeder Offenbarungsstelle in den bevorzugten Ausführungsformen des vorgeschlagenen Verfahrens sowie der vorgeschlagenen Vorrichtungen, welche Garn oder gesponnenes Garn umfassen, insbesondere luftgesponnenes Garn, dieses mitumfasst ist.

Es ist zweckmäßig, wenn mittels des Verfahrens eine Entstehung von mehreren Schwachstellen oder mindestens eines Schwachstellenabschnitts des gesponnenen Garns erkannt wird. Hierdurch lassen sich ganze Schwachstellenbereiche oder die Entstehung ganzer Schwachstellenbereiche des gesponnenen Garns identifizieren.

Darüber hinaus ist es bevorzugt, wenn das vorgeschlagene Verfahren dann initialisiert wird, wenn ein Spinnvorgang initialisiert wird, wobei mit dem Spinnvorgang Garn gesponnen werden soll, das durch das Verfahren auf eine Entstehung von wenigstens einer Schwachstelle überwacht werden soll. Hierdurch wird sichergestellt, dass das Garn, welches mittels des Spinnvorgangs gesponnen werden soll, von Beginn an mittels des Verfahrens überwacht wird.

Weiter ist es in bevorzugter Weise denkbar, dass das vorgeschlagene Verfahren so lange wiederholt wird, solange ein Spinnvorgang oder der Spinnvorgang andauert, mit dem Garn gesponnen wird, das mittels des vorgeschlagenen Verfahrens auf eine Entstehung von wenigstens einer Schwachstelle überwacht werden soll. Hierdurch wird eine lückenlose Überwachung des gesponnenen Garns sichergestellt. Dabei ist es nicht ausgeschlossen, dass der Spinnvorgang aufgrund von Wartungsarbeiten oder Reinigungsvorgängen zeitweise unterbrochen wird. Daher ist es bevorzugt, wenn das vorgeschlagene Verfahren während Unterbrechungen des Spinnvorgangs ebenfalls unterbrochen wird. Hierdurch können Ressourcen, insbesondere Energie, zur Ausführung des vorgeschlagenen Verfahrens eingespart werden.

Darüber hinaus ist es bevorzugt, wenn das vorgeschlagene Verfahren beendet wird, wenn der Spinnvorgang beendet wird, mit dem Garn gesponnen wird, das mittels des vorgeschlagenen Verfahrens auf eine Entstehung von wenigstens einer Schwachstelle überwacht werden soll. Hierdurch können Ressourcen, insbesondere Energie, zur Ausführung des Verfahrens eingespart werden.

Zum Unterbrechen und/oder Beenden des vorgeschlagenen Verfahrens kann es vorgesehen sein, dass die für das Verfahren eingesetzten Komponenten wie Sensoren und/oder Lichtquellen wie beispielsweise Leuchtdioden abgeschaltet werden.

Bei der wenigstens einen Schwachstelle handelt es sich vorzugsweise um eine Stelle des Garns, welche eine Festigkeit, insbesondere Zugfestigkeit aufweist, die unter einem vorgegebenem Festigkeitsschwellwert liegt. Unter der Schwachstelle ist vorzugsweise ein strukturelles Defizit zu verstehen, welches insbesondere dadurch gekennzeichnet ist, dass es dem Garn an Umwindungsfasern fehlt oder die Umwindungsfasern nicht in ausreichender Anzahl vorhanden sind. Umwindungsfasern, die einen Kern eines Garns umgeben, sind insbesondere für die Festigkeit des luftgesponnenen Garns maßgeblich.

Es ist bevorzugt, wenn der mindestens eine Erfassungswert, welcher aus einer optischen Erfassung des Garns resultiert, mittels mindestens eines optischen Sensors erfasst wurde oder erfasst wird. Mit anderen Worten ist es bevorzugt, wenn im Rahmen des Verfahrens der mindestens eine Erfassungswert durch mindestens einen optischen Sensor für den Verfahrensschritt des Empfangens bereitgestellt wurde oder wird. Bei dem mindestens einen optischen Sensor handelt es sich bevorzugterweise um mindestens einen Helligkeitssensor oder um mindestens eine Foto-Diode. Auf diese Weise lässt sich verhältnismäßig einfach und kostengünstig mindestens ein Erfassungswert ermitteln, der für eine Helligkeit des Garns spezifisch ist. Vorzugsweise handelt es sich um genau einen, zwei oder drei solcher Sensoren. Auf den mindestens einen optischen Sensor wird im Rahmen einer erfindungsgemäßen Überwachungsvorrichtung näher eingegangen. Bei den optischen Sensoren kann es sich darüber hinaus um einen oder zwei Remissions-Dioden und insbesondere um eine Abschattungsdiode handeln.

Es ist ferner nach einer bevorzugten Ausführungsform möglich, dass das Auswerten des mindestens einen Erfassungswerts den folgenden Schritt umfasst: Vergleichen des mindestens einen Erfassungswerts, der insbesondere einem Helligkeitswert und vorzugsweise einem Helligkeitsgrad des Garns entspricht, mit mindestens einem Referenzwert, vorzugsweise einem Helligkeitsreferenzwert, um die Unregelmäßigkeit auf Basis einer Abweichung des mindestens einen Erfassungswerts zum mindestens einen Referenzwert festzustellen, wobei vorzugsweise die Entstehung der wenigstens einen Schwachstelle auf Basis einer quantitativen Auswertung der Abweichung erkannt wird, wobei bevorzugt hierdurch die wenigstens eine Schwachstelle in der Form eines strukturellen Defizits des Garns erkannt wird. Insbesondere kann hierbei der mindestens eine Referenzwert eine Referenz-Lichtmenge spezifizieren, welche mindestens von dem Garn remittiert werden soll.

Grundsätzlich ist es bevorzugt, wenn es sich bei dem Helligkeitswert und/oder dem Helligkeitsreferenzwert um Remissionswerte des Garns handelt. Hierdurch lässt sich die Erkennung der Entstehung von wenigstens einer Schwachstelle des gesponnenen Garns genauer durchführen als beispielsweise mittels einer Durchmessererfassung des Garns, die lediglich auf ermittelten Abschattungswerten basiert. Es ist jedoch bevorzugt, wenn eine Durchmessererfassung des Garns, die auf ermittelten Abschattungswerten basiert, beim Auswerten des mindestens einen Erfassungswerts, insbesondere Helligkeitswerts, berücksichtigt wird. Auf diese Weise können insbesondere Störeinflüsse, die den mindestens einen Erfassungswert, insbesondere Helligkeitswert, verfälschen oder manipulieren könnten, kompensiert werden.

Unabhängig hiervon, ist es auch grundsätzlich bevorzugt, wenn Werte jeglicher Art, welche durch das vorgeschlagene Verfahren genutzt werden, beispielsweise der mindestens eine Erfassungswert oder der mindestens eine Referenzwert, in Form von Daten, digitalen Daten oder digitalen Signalen vorliegen. Ferner ist es bevorzugt, wenn die besagten Werte derart vorliegenden, dass sie von einer Vorrichtung für Datenverarbeitung verarbeitet werden können. Das vereinfacht die Verarbeitung der betreffenden Werte. Ferner ist das Verfahren besonders zuverlässig durch eine Vorrichtung zur Datenverarbeitung, insbesondere einen Computer, ausführbar. Bei dem mindestens einen Erfassungswert, aber auch insbesondere bei dem Referenzwert kann es sich um Messwerte oder Messdaten handeln. Auch lassen sich die besagten Werte auf diese Weise einfacher dokumentieren. So kann beispielsweise eine Spule gesponnenes Garn zusammen mit einer Dokumentation von Erfassungswerten, insbesondere in Form von Daten, bereitgestellt werden, um die Eigenschaften des Garns nachzuweisen oder die Eigenschaften des Garns bei dessen Weiterverarbeitung insbesondere mittels einer Textilmaschine zu berücksichtigen.

Ferner ist es nach einer bevorzugten Ausführungsform möglich, dass die Unregelmäßigkeit in Bezug auf die Helligkeit des Garns festgestellt wird, wenn der mindestens eine Helligkeitswert des Garns oder ein davon abgeleiteter Wert den mindestens einen Helligkeitsreferenzwert unterschreitet. Mit einem von dem Helligkeitswert abgeleiteten Wert ist vorzugsweise gemeint, dass sich der abgeleitete Wert beispielsweise aus einer Weiterverarbeitung des mindestens einen Helligkeitswerts mittels eines statistischen Verfahrens und/oder einer mathematischen Formel ergibt. Hierbei können auch weitere Werte, wie beispielsweise ein Wert, der charakteristisch für den Durchmesser des Garns ist, berücksichtigt werden. Auch Kompensationsfaktoren, beispielsweise zur Kompensation von Verunreinigungen des Garns oder Offset-Werte können so berücksichtigt werden. Im Fall, dass weitere Werte berücksichtigt werden, ist es bevorzugt, wenn diese Werte ebenfalls in Form von Erfassungswerten empfangen werden. Mittels des abgeleiteten Werts können verschiedene Störeinflüsse ausgeschlossen oder zumindest teilweise, vorzugsweise größtenteils oder vollständig, kompensiert werden und die Genauigkeit der Werte erhöht oder standardisiert werden. Es ist ferner bevorzugt, dass keine Entstehung einer Schachstelle des Garns erkannt wird, wenn der mindestens eine Helligkeitswert des Garns oder ein davon abgeleiteter Wert den mindestens einen Helligkeitsreferenzwert nicht unterschreitet. Darüber hinaus ist es bevorzugt, wenn in einem solchen Fall das Verfahren, wiederbeginnend mit dem Empfangen mindestens eines, insbesondere weiteren, Erfassungswerts so lange wiederholt wird, solange ein dazugehöriger Spinnvorgang durchgeführt und/oder keine Schwachstelle des Garns erkannt wird. Es ist bevorzugt, wenn der Helligkeitswert des Garns sinkt, wenn die Helligkeit des Garns sinkt. Alternativ ist es nach einer bevorzugten Ausführungsform denkbar, dass sich der Helligkeitswert umgekehrt proportional zur Helligkeit des Garns verhält, also sinkt, wenn die Helligkeit des Garns abnimmt. Dies kommt auf den jeweiligen Anwendungsfall an.

Ferner ist es bevorzugt, wenn die Unregelmäßigkeit in Bezug auf die Helligkeit des Garns erst festgestellt wird, wenn der mindestens eine Helligkeitswert des Garns oder ein davon abgeleiteter Wert den mindestens einen Helligkeitsreferenzwert um mindestens 0,5 %, 1 %, 1,5 %, 2 %, 2,5 %, 3 %, 3,5 %, 4 %, 4,5 % oder 5 % unterschreitet.

Weiter ist es nach einer bevorzugten Ausführungsform denkbar, dass der mindestens eine Erfassungswert, insbesondere Helligkeitswert, wenigstens einen der folgenden Werte umfasst:
- mehrere Helligkeitswerte,
- einen Helligkeitswertverlauf,
- eine Helligkeitswertänderung,
- eine Helligkeitswertänderungsrate,
und dass vorzugsweise der mindestens eine Referenzwert, insbesondere Helligkeitsreferenzwert, wenigstens einen der folgenden Referenzwerte umfasst:
- mehrere Helligkeitsreferenzwerte,
- einen Helligkeitsreferenzwertverlauf,
- eine Helligkeitsreferenzwertänderung,
- eine Helligkeitsreferenzwertänderungsrate.

Die jeweilige bevorzugte Verwendung der oben genannten Erfassungswerte oder Referenzwerte führt in ihrer jeweils genannten Reihenfolge zu einer genaueren Erkennung der Entstehung der wenigstens einen Schwachstelle gesponnenen Garns, während sich der Aufwand zur Durchführung des Verfahrens dadurch lediglich leicht erhöht.

Es ist bevorzugt, wenn sich die Helligkeitswertänderung auf einen zuvor, insbesondere unmittelbar zuvor, erfassten Helligkeitswert bezieht. Damit ist vorzugsweise gemeint, dass ein Unterschied zwischen einem empfangenen Helligkeitswert und einem zuvor empfangenen Helligkeitswert ermittelt wird. So kann beispielsweise eine maximale Helligkeitswertänderung von 0,5 %, 1 %, 1,5 %, 2 %, 2,5 %, 3 %, 3,5 %, 4 %, 4,5 % oder 5 % zulässig sein. Je geringer die maximal zulässige Helligkeitswertänderung ist, desto frühzeitiger sind die Entstehungen von Schwachstellen des Garns mittels des Verfahrens erkennbar.

Darüber hinaus ist es bevorzugt, wenn der Helligkeitswertverlauf aus mehreren Helligkeitswerten besteht und/oder der Helligkeitsreferenzwertverlauf aus mehreren Helligkeitsreferenzwerten besteht. Der Helligkeitswertverlauf bezieht sich vorzugsweise auf Helligkeitswerte, die nacheinander über eine Länge des Garns erfasst werden.

Außerdem ist es zweckmäßig, wenn mittels des mindestens einen Erfassungswerts, insbesondere Helligkeitswerts, vorzugsweise mittels einer statistischen Methode oder mathematischen Formel ein Trend, vorzugsweise ein Trend zukünftiger Helligkeitswerte, abgeleitet wird, durch den eine Entstehung von mindestens einer Schwachstelle des Garns erkannt werden kann. Hierdurch wird eine frühzeitige Erkennung von mindestens einer Schwachstelle des Garns sichergestellt.

Außerdem ist es nach einer bevorzugten Ausführungsform denkbar, dass der mindestens eine Erfassungswert, vorzugsweise Helligkeitswert, einem gemittelten Wert und vorzugsweise der mindestens eine Referenzwert, insbesondere Helligkeitsreferenzwert, einem gemittelten Wert entspricht. Vorzugsweise ist der gemittelte Wert oder sind die gemittelten Werte über eine definierte Länge des gesponnenen Garns oder über eine definierte Dauer des Spinnvorgangs gemittelt. Durch die Verwendung gemittelter Werte lassen sich kurzzeitige Schwankungen der Erfassungswerte, welche zu einer Fehlerkennung einer Entstehung mindestens einer Schwachstelle des Garns führen könnten, weitestgehend ausschließen. Bevorzugterweise wird über eine Länge von 50, 100, 200 oder 250 Metern des Garns ein Mittelwert gebildet. Bei diesen Längen hat sich herausgestellt, dass Schwachstellen mit einer tolerierbaren Zuverlässigkeit detektierbar sind. Umso größer aber die Länge, über die gemittelt wird, ist, desto geringer ist das Risiko einer Fehlerkennung. So lässt sich nach einer alternativ bevorzugten Ausführungsform der Mittelwert auch über Längen von größer als 250 Metern, in bevorzugter Weise in einem Bereich von mehr als 250 Metern bis bevorzugt einschließlich 1.000 Meter mit einem geringen Risiko der Fehlerkennung bilden. Längen weit über 1.000 Meter wirken sich allerdings nachteilig auf die Produktivität aus. Außerdem lassen sich somit Produktionsschwankungen während des Spinnvorgangs, die nicht zwangsläufig zur Entstehung von Schwachstellen des Garns führen, kompensieren.

Auch ist es nach einer bevorzugten Ausführungsform möglich, dass eine Verunreinigung, insbesondere umfassend Fremdpartikel und/oder Fremdfasern, des Garns beim Auswerten berücksichtigt wird, um einen Einfluss der Verunreinigung auf den mindestens einen Erfassungswert zu kompensieren, wobei hierzu vorzugsweise eine Verunreinigungskalibrierung stattfindet, wenn das Spinnen des Garns gestoppt wird. Unter Fremdfasern sind vorzugsweise Fasern zu verstehen, die nicht zur Ausbildung des gesponnenen Garns verwendet werden. Mit der Berücksichtigung der Verunreinigung beim Auswerten wird sichergestellt, dass eine solche Verunreinigung nicht zu einer Fehlerkennung einer Entstehung von wenigstens einer Schwachstelle des gesponnenen Garns führt. Versuche haben ergeben, dass insbesondere ein Drittel der Helligkeitswertänderung auf Verunreinigungen und zwei Drittel der Helligkeitswertänderungen auf eine Entstehung von wenigstens einer Schwachstelle gesponnenen Garns entfällt. Vorzugsweise wird dieses Verhältnis bei der Berücksichtigung der Verunreinigung beim Auswerten berücksichtigt. Alternativ sind auch andere Verhältnisse, wie beispielsweise ein Viertel zu drei Viertel, Helligkeitswertänderung durch Verunreinigung zu Helligkeitswertänderung durch eine Entstehung von wenigstens einer Schwachstelle gesponnenen Garns, denkbar. Eine Verunreinigungskalibrierung führt vorzugsweise zu einem verbesserten Ergebnis als eine bloße Annahme eines derartigen Verhältnisses, die auf Erfahrungswerten basieren kann. Die Verunreinigungskalibrierung umfasst beispielsweise einen Vergleich zwischen mindestens einem Erfassungswert vor einer Reinigung und einem Erfassungswert nach einer Reinigung, vorzugsweise für denselben Garnstellenbereich oder denselben Garnabschnitt. Durch einen solchen Vergleich kann der Einfluss der zuvor bestehenden Verunreinigung auf den Erfassungswert bestimmt werden und für das Fortführen des Verfahrens zur Kompensation des Einflusses der Verunreinigung auf den mindestens einen Erfassungswert, insbesondere Helligkeitswert, verwendet werden.

Darüber hinaus ist es nach einer bevorzugten Ausführungsform denkbar, für die mittels der Verunreinigungskalibrierung ermittelte notwendige Kompensation der Verunreinigung einen Offsetwert zu berücksichtigen, der vorzugsweise dynamische Effekte während des Spinnvorgangs berücksichtigt. Unter dem Stoppen des Spinnens des Garns ist vorzugsweise eine Unterbrechung des Spinnvorgangs zu verstehen, mittels dem das mit dem vorgeschlagenen Verfahren zu überwachende Garn gesponnen wird. Mit Unterbrechung ist bevorzugterweise gemeint, dass nach der Verunreinigungskalibrierung der Spinnvorgang fortgesetzt wird oder fortgesetzt werden kann.

Weiter ist es nach einer bevorzugten Ausführungsform denkbar, dass das Erkennen der Entstehung der wenigstens einen Schwachstelle zeitlich vor einer vollständigen Ausbildung der wenigstens einen Schwachstelle erfolgt, wobei das Erkennen der Entstehung der wenigstens einen Schwachstelle vorzugsweise in der Form einer Erkennung von fehlenden oder fehlerhaft liegenden Umwindungsfasern des Garns erfolgt. Umwindungsfasern können für die Festigkeit luftgesponnenen Garns maßgeblich sein. Vorzugsweise haben die Umwindungsfaser einen Einfluss auf die Helligkeit des Garns und somit auf den mindestens einen Erfassungswert, insbesondere Helligkeitswert. Indem das Erkennen der Entstehung der wenigstens einen Schwachstelle des Garns zeitlich vor einer vollständigen Ausbildung der wenigstens einen Schwachstelle des Garns erfolgt, werden Probleme bei der Weiterverarbeitung, wie beispielsweise größere Stillstandzeiten und Mehrkosten, vermieden.

Es ist weiter möglich, dass das Verfahren nach einer bevorzugten Ausführungsform den folgenden Schritt umfasst:
- Initiieren einer Reaktion auf das Erkennen der Entstehung der wenigstens einen Schwachstelle, wobei hierzu eine Ausgabe generiert wird, die eine, insbesondere pneumatische, Reinigung einer Spinndüse der Spinnmaschine und/oder ein Beenden oder Unterbrechen eines Spinnvorgangs der Spinnmaschine, insbesondere nach mindestens einer vorangegangenen Reinigung einer Spinndüse oder der Spinndüse der Spinnmaschine, und/oder eine Wartung der Spinnmaschine, insbesondere nach mindestens einer vorangegangenen Reinigung einer Spinndüse oder der Spinndüse der Spinnmaschine, veranlasst, wenn die Entstehung wenigstens einer weiteren Schwachstelle des Garns erkannt wird.

Unter der Reinigung ist insbesondere ein automatisierter Reinigungsvorgang, vorzugsweise mechanisch oder pneumatisch, der Spinnvorrichtung zu verstehen. Mittels der Reinigung wird oftmals eine Ursache für die Entstehung mindestens einer Schwachstelle des gesponnenen Garns behoben. Mit dem Unterbrechen des Spinnvorgangs der Spinnmaschine ist vorzugsweise eine Unterbrechung des Spinnvorgangs an lediglich einer Spinnstelle der Spinnmaschine zu verstehen, an der die Entstehung der wenigstens einen Schwachstelle des Garns erkannt wurde. Hierdurch können, im Fall, dass die Spinnmaschine mehrere Spinnstellen aufweist, die Spinnvorgänge der Spinnstellen, an denen eine solche Entstehung nicht erkannt wurde, fortgesetzt werden. Dies trägt zu einem besonders effektiven Produktionsprozess bei. Vorzugsweise wird bei einer Unterbrechung des Spinnvorgangs eine oder die Verunreinigungskalibrierung durchgeführt. Sollte innerhalb eines bestimmten Zeitraums nach der Reinigung oder nach einer erneuten Reinigung der Spinnvorrichtung wie beispielsweise einer Spinndüse oder eines Spinnrotors, während der Fortführung des Spinnvorgangs, eine weitere Entstehung mindestens einer Schwachstelle des luftgesponnenen Garns erkannt werden, so wird angenommen, dass die Reinigung der Spinnvorrichtung die Entstehung von Schwachstellen nicht abgestellt hat und auch eine erneute Reinigung die Entstehung von Schwachstellen nicht abstellen wird. Daher ist es bevorzugt, dass in einem solchen Fall eine Wartung der Spinnmaschine, insbesondere der betroffenen Spinnstelle der Spinnmaschine, veranlasst wird. Hierfür wird vorzugsweise eine Meldung generiert werden, die beispielsweise auf einem Bildschirm eine Nachricht mit der Aufforderung zur Wartung der Spinnmaschine oder der betroffenen Spinnstelle ausgibt oder die beispielsweise mittels einer definierten Signalfarbe einer Signallampe an der Spinnstelle zur Anzeige gelangt. Nach der Reinigung oder Wartung wird vorzugsweise eine Fortsetzung des Spinnvorgangs der Spinnmaschine, insbesondere der betroffenen Spinnstelle der Spinnmaschine, initiiert, wobei vorzugweise das vorgeschlagene Verfahren, wiederbeginnend mit dem Empfangen von mindestens einem Erfassungswert, ebenfalls initiiert wird.

Darüber hinaus ist es nach einer bevorzugten Ausführungsform denkbar, dass bei dem vorgeschlagenen Verfahren eine Kontrolle von Spinndruck des Spinnvorgangs und/oder Unterdruck einer Absaugung vorgesehen ist. Eine derartige Absaugung unterstützt vorzugsweise die Reinigung des Garns. Hierfür ist es bevorzugt, wenn in Reaktion auf die Erkennung der Entstehung der wenigstens einen Schwachstelle des Garns eine Veränderung der Absaugleistung der Absaugung und/oder eine Veränderung des Spinndrucks des mittels des vorgeschlagenen Verfahrens zu überwachenden Spinnvorgangs erfolgt. Hiermit ist es möglich, die Entstehung der wenigstens einen Schwachstelle des Garns zu beseitigen oder ihr entgegenzuwirken. Darüber hinaus ist es denkbar, dass mittels des mindestens einen Empfangswerts eine Beschädigung oder ein Verschleiß der Spinnvorrichtung erkannt wird, da insbesondere diese Beschädigung oder dieser Verschleiß die Entstehung der mindestens einen Schwachstelle hervorruft. In einem solchen Fall wird vorzugsweise eine Wartung der Spinnmaschine oder der Spinnvorrichtung, insbesondere ein Austausch der Spinndüse bzw. des Spinnrotos mit einer neuwertigen Spinndüse bzw. eines Spinnrotors, veranlasst und insbesondere der Spinnvorgang hierfür unterbrochen.

Ferner ist es nach einer bevorzugten Ausführungsform denkbar, dass das Verfahren die folgenden Schritte umfasst:
- Definieren eines Zeitpunkts eines Spinnvorgangs der Spinnmaschine, ab welchem ein mindestens erster Erfassungswert, insbesondere in der Form eines Remissionswerts des Garns, empfangen werden soll,
- Definieren einer Länge des Garns, für welche der mindestens erste Erfassungswert empfangen werden soll,
- Initiieren des Empfangs des mindestens ersten Erfassungswerts ab dem definierten Zeitpunkt des Spinnvorgangs und für die definierte Länge des Garns, um aus dem empfangenen, mindestens ersten Erfassungswert den mindestens einen Referenzwert zu bilden, vorzugsweise durch eine Mittelwertbildung,
- Initiieren des Empfangs von mindestens einem weiteren Erfassungswert für den weiteren Spinnvorgang, insbesondere wiederholt für die oder eine weitere definierte Länge des Garns, um aus dem mindestens einem weiteren Erfassungswert zumindest einen aktuellen Erfassungswert zu erhalten, vorzugsweise durch eine Mittelwertbildung, wobei das Erkennen der Entstehung der wenigstens einen Schwachstelle des Garns auf Basis eines Vergleichs des aktuellen Erfassungswerts mit dem mindestens einen Referenzwert durchgeführt wird, und die Entstehung der wenigstens einen Schwachstelle des Garns vorzugsweise dann erkannt wird, wenn der aktuelle Erfassungswert von dem Referenzwert mit einem definierten Betrag abweicht.

Auf diese Weise wird zu Beginn des vorgeschlagenen Verfahrens ein Referenzwert geschaffen, der dem weiteren Verlauf des Verfahrens dient. Ferner werden durch die Bildung der Mittelwerte Produktionsschwankungen während des Spinnvorgangs, die sonst zu Fehlerkennungen der Entstehung von Schwachstellen des gesponnenen Garns führen könnten, kompensiert.

Bei der Abweichung kann es sich beispielsweise um eine Unterschreitung oder Überschreitung, welche als nicht tolerierbar definiert ist, handeln. Der Betrag der Abweichung kann in bevorzugter Weise für jeweilige Garnpartien festgelegt werden als auch variabel einstellbar sein. Weiterhin bevorzugt kann der Betrag der Abweichung aus einem Wissensspeicher abrufbar sein, wobei der Wissensspeicher wenigstens einen auslesbaren und insbesondere weiterhin überschreibbaren Betrag für die Abweichung in Abhängigkeit der zu produzierenden Garnpartie und insbesondere zusätzlich einer Qualitätsanforderung an das zu spinnende Garn bzw. der herzustellenden Garnpartie, welche beispielsweise von einem Kunden vorgebbar ist, umfasst. So lassen sich kundenspezifische Garnpartien hinsichtlich der Garnqualität herstellen.

Ebenfalls Gegenstand der Erfindung ist nach einem weiteren Aspekt eine Vorrichtung zur Datenverarbeitung, die Mittel zur Ausführung des vorgeschlagenen Verfahrens umfasst. Damit bringt die vorgeschlagene Vorrichtung zur Datenverarbeitung die gleichen Vorteile mit sich, wie sie ausführlich mit Bezug auf vorgeschlagene Verfahren nach einer der bevorzugten Ausführungsformen beschrieben worden sind. Die Vorrichtung zur Datenverarbeitung kann in ein Steuergerät für eine Spinnmaschine oder einer Spinnstelle oder in ein der Spinnmaschine übergeordnetes Steuergerät integriert sein. Es ist bevorzugt, wenn das Steuergerät dazu ausgebildet ist, Einfluss auf den Betrieb der Spinnmaschine zu nehmen. Im Besonderen ist es aber bevorzugt, wenn das Steuergerät Bestandteil der Spinnmaschine oder Spinnstelle ist. Damit kann die Kompaktheit erhöht werden, da keine zum Steuergerät separate Vorrichtung zur Datenverarbeitung vorgesehen sein muss.

Als der Computer kann eine Datenverarbeitungsvorrichtung, beispielsweise die vorgeschlagene Vorrichtung, vorgesehen sein, welche das Computerprogramm ausführt. Der Computer kann wenigstens einen Prozessor zur Ausführung des Computerprogramms aufweisen. Auch kann ein nicht-flüchtiger Datenspeicher vorgesehen sein, in welchem das Computerprogramm hinterlegt und von welchem das Computerprogramm durch den Prozessor zur Ausführung ausgelesen werden kann. Ferner kann das Computerprogramm auch direkt in einer Maschinensoftware der Maschine integriert sein.

Ebenfalls ist es nach einer bevorzugten Ausführungsform denkbar, dass der Computer zumindest einen integrierten Schaltkreis wie einen Mikroprozessor oder eine anwendungsspezifische integrierte Schaltung (ASIC) oder ein anwendungsspezifisches Standardprodukt (ASSP) oder einen digitalen Signalprozessor (DSP) oder einen Field Programmable Gate Array (FPGA) oder dergleichen umfasst. Der Computer kann ferner wenigstens eine Schnittstelle zum Datenaustausch, z. B. eine Ethernet-Schnittstelle oder eine Schnittstelle für LAN (Local Area Network) oder WLAN (Wireless Local Area Network) oder System-on-a-Chip (SoC) oder eine andere Funkschnittstellen wie Bluetooth oder Nahfeldkommunikation (NFC) aufweisen. Ferner kann der Computer als ein oder mehrere Steuergeräte, d. h. auch als ein System aus Steuergeräten, ausgeführt sein. Der Computer kann beispielsweise auch in einer Cloud und/oder als ein Server vorgesehen sein, um über die Schnittstelle die Datenverarbeitung für eine lokale Anwendung zur Verfügung zu stellen. Auch ist es möglich, dass der Computer als ein mobiles Gerät, wie ein Smartphone, ausgeführt ist.

Ebenfalls Gegenstand der Erfindung nach einem weiteren Aspekt ist eine Überwachungsvorrichtung, die die vorgeschlagene Vorrichtung zur Datenverarbeitung umfasst, wobei es sich bei der Überwachungsvorrichtung insbesondere um einen Garnreiniger handelt. Auf diese Weise lässt sich die Funktion, die durch das vorgeschlagene Verfahren geschaffen wird, äußerst einfach in eine bestehende Vorrichtung, insbesondere einen Garnreiniger, integrieren. Außerdem können zur Ausführung des vorgeschlagenen Verfahrens bereits im Garnreiniger vorgesehene optische Sensoren, die insbesondere zur Identifikation von Verunreinigungen des Garns dienen, genutzt werden. Außerdem bringt die vorgeschlagene Überwachungsvorrichtung die gleichen Vorteile mit sich, wie sie ausführlich mit Bezug auf das vorgeschlagene Verfahren nach einer der bevorzugten Ausführungsformen beschrieben worden sind.

Es ist außerdem möglich, dass die Überwachungsvorrichtung eine künstliche Lichtquelle, insbesondere eine Infrarot-Leuchtdiode, zur Bestrahlung des Garns mit Licht, insbesondere Infrarot-Licht, umfasst, und dass die Überwachungsvorrichtung zumindest oder genau eine oder zwei optische Sensoren, vorzugsweise Foto-Dioden, insbesondere Remissions-Dioden, zur Erfassung des durch das Garn reflektierten Lichts der Lichtquelle umfasst, die benachbart zur Lichtquelle angeordnet sind, und dass die Überwachungsvorrichtung vorzugsweise einen dritten optischen Sensor, vorzugsweise eine dritte Foto-Diode, umfasst, die von der Lichtquelle aus gesehen hinter dem Garn angeordnet ist und zur Erfassung einer Abschattung, die sich durch die Anordnung des Garns im Verhältnis zur Lichtquelle sowie dem Licht der Lichtquelle ergibt, umfasst. Bei den optischen Sensoren handelt es sich bevorzugterweise um Sensoren zur Messung von Helligkeit, insbesondere Helligkeitssensoren. Das Garn verläuft vorzugsweise durch einen Messraum der Überwachungsvorrichtung, insbesondere des Garnreinigers, wobei der Messraum die Lichtquelle mit dem mindestens einen optischen Sensor zur Erfassung des durch das Garn reflektierten Lichts der Lichtquelle auf der einen Seite von dem optischen Sensor zur Erfassung einer Abschattung auf der anderen Seite trennt. Die optischen Sensoren und die Lichtquelle befinden sich vorzugsweise in einer Ebene, die senkrecht zu einer Förderrichtung des Garns oder zu einer Längsachse des Garns verläuft. Darüber hinaus ist es bevorzugt, wenn die Lichtquelle zur Bestrahlung des Garns mittels Lichts und der mindestens eine optische Sensor zur Erfassung des durch das Garn reflektierten Lichts derart aufeinander, und insbesondere auf den Garn, abgestimmt sind, dass durch den mindestens einen optischen Sensor die Helligkeit des Garns in Reaktion auf die Bestrahlung des Garns mit Licht der Lichtquelle ermittelbar ist.

Ebenfalls Gegenstand der Erfindung nach einem weiteren Aspekt ist eine Spinnmaschine, insbesondere Luftspinnmaschine, die mindestens eine vorgeschlagene Überwachungsvorrichtung umfasst. Damit bringt die vorgeschlagene Spinnmaschine die gleichen Vorteile mit sich, wie sie ausführlich mit Bezug auf ein vorgeschlagenes Verfahren nach einer der bevorzugten Ausführungsformen beschrieben worden sind. Es ist bevorzugt, dass die Spinnmaschine in üblich angeordneter Weise mehrere Spinnstellen zum Spinnen von gesponnenem, insbesondere luftgesponnenem, Garn aufweist. Hierbei ist es bevorzugt, wenn jede Spinnstelle eine vorgeschlagene Überwachungsvorrichtung umfasst. Somit sind mittels des vorgeschlagenen Verfahrens und der vorgeschlagenen Überwachungsvorrichtung mehrere parallele Spinnvorgänge der Spinnmaschine derart überwachbar, dass eine Entstehung von wenigstens einer Schwachstelle eines gesponnenen, insbesondere luftgesponnenen, Garns erkennbar ist. Dies führt zu einer besonders produktiven Spinnmaschine. Ferner ist es bevorzugt, wenn die Spinnmaschine das Steuergerät umfasst.

Ebenfalls Gegenstand der Erfindung nach einem weiteren Aspekt ist ein Computerprogramm, welches Befehle umfasst, die bei der Ausführung des Computerprogramms durch einen Computer diesen veranlassen, das vorgeschlagene Verfahren auszuführen. Ebenfalls Gegenstand der Erfindung nach einem weiteren Aspekt ist ein Computerprogrammprodukt, umfassend Befehle, die bei der Ausführung des Computerprogrammes durch einen Computer diesen veranlassen, das vorgeschlagene Verfahren auszuführen. Damit bringt das vorgeschlagene Computerprogramm als auch das vorgeschlagene Computerprogrammprodukt die gleichen Vorteile mit sich, wie sie ausführlich mit Bezug auf ein vorgeschlagenes Verfahren nach einer der bevorzugten Ausführungsformen beschrieben worden sind.

Ebenfalls Gegenstand der Erfindung nach einem weiteren Aspekt ist ein computerlesbares Medium, auf dem das vorgeschlagene Computerprogramm abgespeichert ist. Das computerlesbare Medium ist vorzugsweise als ein Speichermedium, beispielsweise als ein Datenspeicher wie eine Festplatte und/oder ein nicht-flüchtiger Speicher und/oder eine Speicherkarte ausgebildet. Das Speichermedium kann z. B. in dem Computer oder in das Steuergerät integriert sein. Damit bringt das vorgeschlagene computerlesbare Medium die gleichen Vorteile mit sich, wie sie ausführlich mit Bezug auf ein vorgeschlagenes Verfahren nach einer der bevorzugten Ausführungsformen beschrieben worden sind.

Darüber hinaus kann das vorgeschlagene Verfahren, insbesondere grundsätzlich, auch als ein computerimplementiertes Verfahren ausgeführt sein.

### Figurenbeschreibung

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein.

Es zeigen:
- Fig. 1: schematisch ein Ausführungsbeispiel eines Verfahrens,
- Fig. 2: schematische eine Darstellung eines Helligkeitswertverlaufs,
- Fig. 3: schematisch ein Ausführungsbeispiel einer Spinnmaschine, und
- Fig. 4: schematisch ein Ausführungsbeispiel einer Überwachungsvorrichtung.

In den nachfolgenden Figuren werden für die gleichen technischen Merkmale auch von unterschiedlichen Ausführungsbeispielen die identischen Bezugszeichen verwendet.

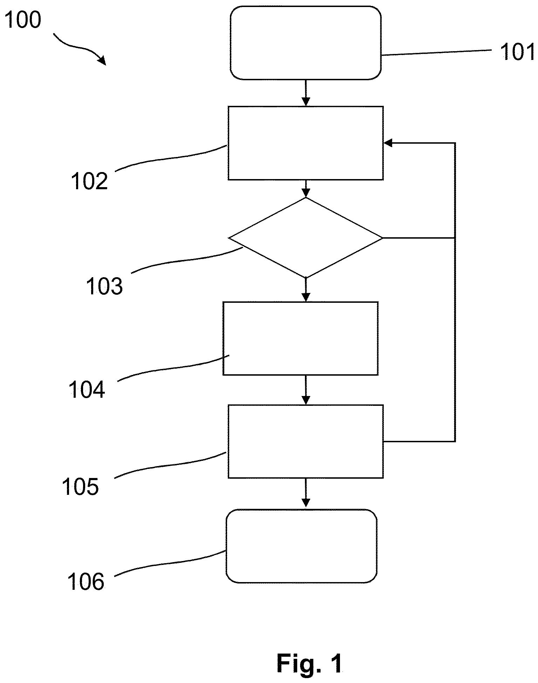

In Figur 1 ist schematisch ein Ausführungsbeispiel eines Verfahrens 100 zur Erkennung einer Entstehung von wenigstens einer Schwachstelle eines luftgesponnenen Garns dargestellt. Unter der Schwachstelle ist vorzugsweise ein strukturelles Defizit zu verstehen, welches dadurch gekennzeichnet ist, dass es dem Garn an Umwindungsfasern fehlt oder diese nicht in ausreichender Anzahl vorhanden sind. Zunächst erfolgt das Initialisieren 101 des Verfahrens 100, welches vorzugsweise zeitgleich mit dem Beginn eines Spinnvorgang einer Spinnmaschine, bei der das Verfahren 100 zum Einsatz kommt, erfolgt.

Als Nächstes erfolgt ein Empfangen 102 von mindestens einem Erfassungswert. Der mindestens eine Erfassungswert ist spezifisch für eine Helligkeit des Garns und wurde vorzugsweise mittels mindestens eines optischen Sensors ermittelt.

Daraufhin erfolgt ein Auswerten 103 des mindestens einen Erfassungswerts, um eine Unregelmäßigkeit in Bezug auf die Helligkeit des Garns festzustellen. Das Auswerten 103 umfasst ein Vergleichen des mindestens einen Erfassungswerts mit mindestens einem Referenzwert, um die Unregelmäßigkeit auf Basis einer Abweichung des mindestens einen Erfassungswerts zum mindestens einen Referenzwert festzustellen. Der mindestens eine Erfassungswert entspricht einem Helligkeitswert des Garns, während der Referenzwert einem Helligkeitsreferenzwert entspricht.

Als nächster Schritt erfolgt ein Erkennen 104 der Entstehung der wenigstens einen Schwachstelle des luftgesponnenen Garns auf Basis der festgestellten Unregelmäßigkeit. Mittels einer derartigen Unregelmäßigkeit lässt sich auf die zu erkennende Schwachstelle schließen. Sollte eine derartige Unregelmäßigkeit festgestellt werden, wird die Entstehung der wenigstens einen Schwachstelle des Garns erkannt. Im Fall, dass keine derartige Unregelmäßigkeit festgestellt wird, wird erkannt, dass keine Schwachstelle entsteht. Mit anderen Worten kann in einem derartigen Fall, in dem keine derartige Unregelmäßigkeit festgestellt wird, davon ausgegangen werden, dass schwachstellenfreies Garn vorliegt. Wenn keine Schwachstelle des Garns erkannt wird, erfolgt vorzugsweise eine Wiederholung des Verfahrens, wiederbeginnend mit dem Empfangen 102 von mindestens einem weiteren Erfassungswert, solange Garn mittels einer Spinnmaschine luftgesponnen wird.

Wenn jedoch eine Entstehung einer Schwachstelle erkannt wird, dann erfolgt in Reaktion auf das Erkennen 104 der wenigstens einen Schwachstelle vorzugsweise ein Generieren 105 einer Ausgabe, die eine Unterbrechung des Spinnvorgangs der Spinnmaschine sowie eine automatisierte, pneumatische Reinigung einer Spinndüse der Spinnmaschine veranlasst, da mit hoher Wahrscheinlichkeit ausgegangen werden kann, dass eine Verunreinigung der Spinndüse der Spinnmaschine die Entstehung der mindestens einen Schwachstelle hervorgerufen hat. Bei solchen Verunreinigungen handelt es sich meist um Fremdfasern. Nach der Reinigung wird vorzugsweise eine Fortsetzung des Spinnvorgangs der Spinnmaschine initiiert und das Verfahren 100, wiederbeginnend mit dem Empfangen 102 von, insbesondere weiteren, Erfassungswerten wiederholt. Sollte innerhalb eines bestimmten Zeitraums nach der Reinigung oder nach einer erneuten Reinigung der Spinndüse eine weitere Entstehung mindestens einer Schwachstelle des luftgesponnenen Garns erkannt werden, so wird angenommen, dass die Reinigung der Spinndüse die Entstehung der wenigstens einen Schwachstelle des Garns nicht abgestellt hat und auch eine erneute Reinigung die Entstehung der wenigstens einen Schwachstelle nicht abstellen wird. In einem solchen Fall wird ein Unterbrechen des Spinnvorgangs der Spinnmaschine veranlasst. Zudem wird eine Ausgabe generiert, die eine Wartung der Spinnmaschine veranlasst. Beispielsweise kann hierfür eine Meldung generiert werden, die auf einem Bildschirm eine Nachricht mit der Aufforderung zur Wartung der Spinnmaschine erzeugt. Nach der Wartung kann das Verfahren 100, wiederbeginnend mit dem Empfangen 102 von mindestens einem, insbesondere weiteren, Erfassungswert wiederholt werden. Wenn der Spinnvorgang der Spinnmaschine beendet wird, ist es bevorzugt, wenn ebenfalls ein Beenden 106 des Verfahrens 100 erfolgt.

Das Verfahren 100 ist vorzugsweise computerimplementiert. Darüber hinaus ist es bevorzugt, wenn der mindestens eine Erfassungswert und der mindestens eine Referenzwert als, insbesondere digitale, Daten vorliegen.

Außerdem ist es bevorzugt, wenn der mindestens eine Erfassungswert und/oder der mindestens eine Referenzwert als Mittelwert, gemittelt über die Länge des Garns, beispielsweise gemittelt über 100 Meter, vorliegt und/oder vorliegen. Der Referenzwert wird vorzugsweise dadurch gebildet, dass über eine definierte Länge des Garns ein Mittelwert gebildet wird. Eine derartige Bildung des Referenzwerts kann vor der Initialisierung des Verfahrens 100 erfolgen.

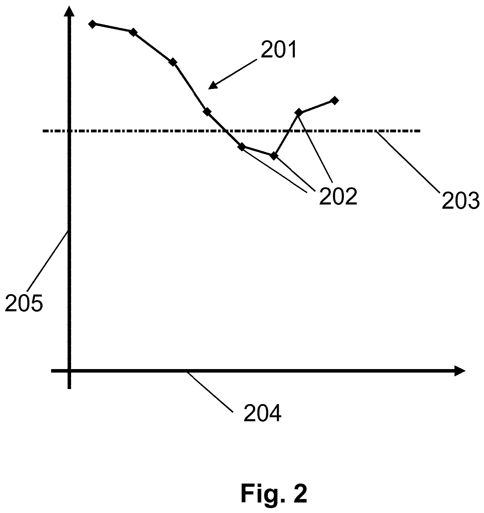

In Figur 2 ist eine schematische Darstellung eines Helligkeitswertverlaufs 201 dargestellt. Die Abszisse 204 stellt hierbei die Länge des luftgesponnenen Garns dar, während die Ordinate 205 quantitativ die Helligkeit einzelner Helligkeitswerte 202 beschreibt. Die einzelnen Helligkeitswerte 202 sind über der Länge des Garns gemittelte Werte und bilden den Helligkeitswertverlauf 201. Ferner ist ein Helligkeitsreferenzwert 203 dargestellt. Wenn der Helligkeitswertverlauf 201 oder mindestens einer der Helligkeitswerte 202 den Helligkeitsreferenzwert 203 unterschreitet, wird die Unregelmäßigkeit in Bezug auf die Helligkeit des Garns festgestellt. In einer weiteren Ausführungsform ist es bevorzugt, wenn der Helligkeitsreferenzwert 203 als Helligkeitsreferenzwertverlauf ausgebildet ist.

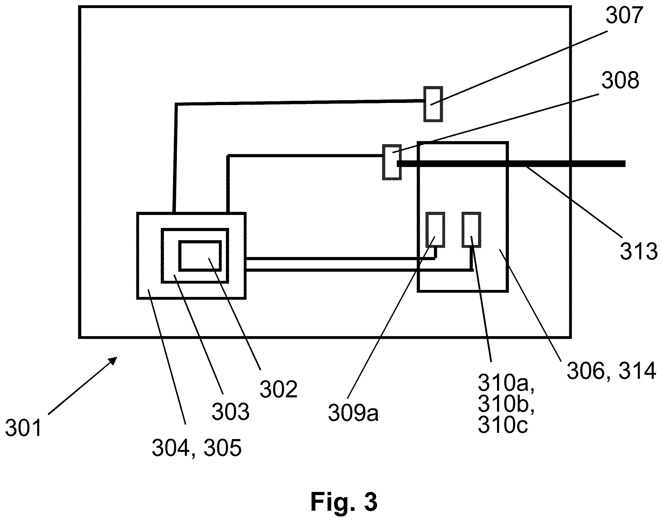

In Figur 3 ist schematisch ein Ausführungsbeispiel einer Spinnmaschine 301 dargestellt. Bei der Spinnmaschine 301 handelt sich um eine Luftspinnmaschine. Die Spinnmaschine 301 ist dazu ausgebildet, luftgesponnenes Garn 313 herzustellen. Hierfür umfasst die Spinnmaschine 301 eine Spinndüse 308, eine Absaugung 307 sowie eine Überwachungsvorrichtung 306. Die Überwachungsvorrichtung 306 ist dazu ausgebildet, das Verfahren 100 auszuführen. Bei der Überwachungsvorrichtung 306 handelt es sich bevorzugterweise um einen Garnreiniger 314, der ferner dazu ausgebildet ist, Verunreinigungen des Garns zu erkennen. Der Garnreiniger 314 umfasst eine künstliche Lichtquelle 309a, welche als Leuchtdiode 309b, insbesondere als Infrarot-Leuchtdiode 309c, ausgebildet ist. Außerdem umfasst die Überwachungsvorrichtung 306 mindestens einen, vorzugsweise drei optische Sensoren 310a, 310b, 310c. Der mindestens eine ist, die vorzugsweise drei optischen Sensoren 310a, 310b, 310c sind, vorzugsweise als Helligkeitssensor oder als Foto-Diode ausgebildet. Bei den ersten beiden optischen Sensoren handelt es sich vorzugsweise um Remissions-Dioden, während es sich bei dem dritten optischen Sensor um eine Abschattungs-Diode handelt.

Die Leuchtdiode 309b ist durch eine Vorrichtung zur Datenverarbeitung 304, insbesondere einen Computer, betreibbar. Die erfassten Werte der optischen Sensoren 310a, 310b, 310c sind kabelgebunden an die Vorrichtung zur Datenverarbeitung 304 übertragbar. Die Vorrichtung zur Datenverarbeitung 304 ist vorzugsweise in ein Steuergerät 305 der Spinnmaschine 301 integriert. Mittels des Steuergeräts 305 ist zudem die Spinnmaschine 301 betreibbar. Die Vorrichtung zur Datenverarbeitung 304 umfasst ferner ein computerlesbares Medium 303, welches vorzugsweise als nichtflüchtiger Speicher ausgebildet ist, und auf dem ein Computerprogramm 302 abgespeichert ist. Das Computerprogramm 302 umfasst Befehle, die bei der Ausführung des Computerprogramms 302 durch die Vorrichtung zur Datenverarbeitung 304, bewirken, dass die Vorrichtung zur Datenverarbeitung 304 das Verfahren 100 ausführt. In der Figur 3 ist die Vorrichtung zur Datenverarbeitung 304, das computerlesbare Medium 303 sowie das Computerprogramm 302 außerhalb der Überwachungsvorrichtung 306 dargestellt, was allerdings nicht zwingend erforderlich, sondern lediglich optional ist.

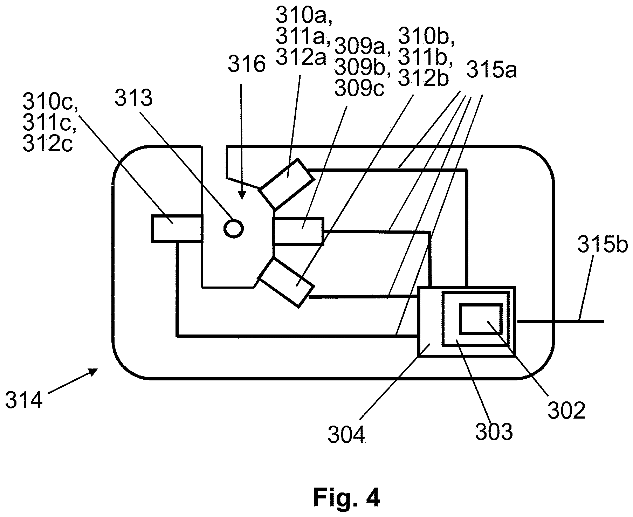

In Figur 4 ist schematisch ein Ausführungsbeispiel der Überwachungsvorrichtung 306, die als Garnreiniger 314 ausgebildet ist, dargestellt. Im Unterschied zur Darstellung aus der Figur 3 ist die Vorrichtung zur Datenverarbeitung 304 mit dem computerlesbaren Medium 303 und dem Computerprogramm 302 in den Garnreiniger 314 integriert. Die Darstellung in Figur 4 zeigt eine Ebene, die durch den Garnreiniger 314 und senkrecht zu einer Förderrichtung des Garns 313 verläuft. Mit anderen Worten verläuft die Ansicht der Figur 4 senkrecht zur Längsachse des Garns 313. Das Garn 313 ist durch einen Messraum 316 des Garnreinigers 314 förderbar. Mittels der künstlichen Lichtquelle 309a, die vorzugsweise als Infrarot-Leuchtdiode 309c ausgebildet ist, wird das durch den Garnreiniger 314 geförderte Garn 313 mittels Lichts, vorzugsweise mittels Infrarot-Lichts, bestrahlt. In Reaktion auf diese Bestrahlung reflektiert das Garn 313 während seiner Förderung das auf ihn gestrahlte Licht in Richtung von zwei optischen Sensoren 310a, 310b, die benachbart zur künstlichen Lichtquelle 309a angeordnet sind und vorzugsweise als Remissions-Dioden ausgebildet sind. Da sich von der künstlichen Lichtquelle 309a aus gesehen hinter dem Garn 313 ein dritter optischer Sensor 310c befindet, wird mittels des dritten optischen Sensors 310c eine Abschattung, die sich aus der Anordnung des dritten optischen Sensors 310c, des Garns 313 und der künstlichen Lichtquelle 309a ergibt, erfasst. Die optischen Sensoren 310a, 310b, 310c sind vorzugsweise als Helligkeitssensoren 311a, 311b, 311c oder als Foto-Dioden 312a, 312b, 312c ausgebildet. Die durch die optischen Sensoren 310a, 310b, 310c erfassten Werte sind mittels Kabel 315a an die Vorrichtung zur Datenverarbeitung 304 übertragbar. Durch ein weiteres Kabels 315b ist es möglich, dass die Vorrichtung zur Datenverarbeitung 304 eine Wartung der Spinnmaschine 301 veranlasst, in dem eine Nachricht generiert und auf einem Monitor angezeigt wird, die zu einer Wartung der Spinnmaschine auffordert.

Im Folgenden wird eine weitere Ausführungsform der Erfindung beschrieben.

Im Messraum des Garnreinigers werden Helligkeitsunterschiede, insbesondere in Form von Remissionswerten, des gesponnenen Garns erfasst. Das wird herkömmlicherweise oft nur dazu genutzt, um Fremdfasern jeder Art durch Änderung der Helligkeit zu erkennen und diese dann im Garn auszureinigen. Es ist eine von Ausführungsvarianten der Erfindung zugrundeliegende Idee, die vom Garnreiniger erfassten Helligkeitsunterschiede zur Früherkennung der Entstehung von Schwachstellen des Garns zu nutzen.

Schwachstellen entstehen üblicherweise dann, wenn sich die für ein luftgesponnenes Garn notwendigen Umwindungsfasern nicht um den Garnkern legen und somit der für den Garn typische Charakter fehlt. Erst die Umwindungsfasern geben dem Garn die notwenige Festigkeit. Eine Störung bei der Bildung der Umwindungsfasern kann somit zwangsläufig zu unerwünschten Schwachstellen im Garn führen.

Eine störungsfreie Bildung der Umwindungsfasern ist grundsätzlich von bestimmten Einflüssen abhängig. Das sind z. B. Spinndruck, störungsfreie Absaugung von Schmutz und Fragmenten, ordnungsgemäße Position der Spinndüse und der dazugehörigen Teile zur Bildung des Fadens bzw. Garns. Weiterhin ist es oft zwingend erforderlich, dass nur unbeschädigte und ordnungsgemäße Teile, insbesondere eine ordnungsgemäße Spinndüse einer Spinnmaschine, im Bereich der Bildung des Fadens zum Einsatz kommen.

Ausführungsvarianten der Erfindung können es ermöglichen, dass negative Einflüsse der vorgenannten Störungen rechtzeitig erkannt werden. Der Garnreiniger kann hierzu beispielsweise die Garnwerte mit einer definierten Abtastung erfassen. Konkret können hierzu Helligkeitsänderungen, insbesondere schmutzkompensiert, stetig erfasst und berechnet werden. Ein sich ändernder Garncharakter kann sich auf die Remission und somit auf die Helligkeit auswirken.

Gibt es im Garnbildungsprozess eine der zuvor genannten Störungen, kann das einen direkten Einfluss auf die notwendigen Umwindungsfasern haben. Fehlende oder nicht um den Faden liegende Umwindungsfasern können gemäß Ausführungsvarianten der Erfindung in der Remission sicher erkannt werden, da diese die Helligkeit des Garns ändern.

Es kann vorgesehen sein, dass die Remissionswerte eines Garns erfasst und über eine bestimmte Länge ein Mittelwert, vorzugsweise Referenzwert, gebildet wird. Dieser Referenzwert kann festgesetzt und abgespeichert werden. Bei dem nun weiter gesponnenen Garn können die Mittelwerte über eine bestimmte Länge, z. B. 100 Meter, gebildet und mit dem Referenzwert verglichen werden. Es ist dann möglich, dass durch eine nun eingestellte Abweichung der Helligkeit vom Referenzwert zum aktuellen Mittelwert einer bestimmten Länge eine Änderung des Garncharakters und somit der Umwindungsfasern festgestellt wird. Die Spinnstelle kann abgestellt werden und auf Fehler überprüft werden. Somit werden Schwachstellen schon in der Entstehung erkannt und nicht erst wenn diese vorhanden sind.

Eine erweiterte Ausführungsform sieht vor, die Entstehung von Schwachstellen auch zu erkennen, wenn farblich inhomogenes oder dunkles Garn gesponnen wird. Hierfür kann eine Infrarot-Leuchtdiode zur Bestrahlung des Garns verwendet werden. Hierdurch wird die Remission und somit die Helligkeit genauso durch den Garncharakter beeinflusst wie mit normalen Lichtquellen.

Entsprechend eines beispielhaften Verfahrensablaufs können gemäß einem ersten Schritt Remissionswerte des Garns ab einem definierten Zeitpunkt des Spinnvorgangs erfasst werden. Sodann kann gemäß einem weiteren Schritt ein Referenzwert, insbesondere ein Mittelwert aus den Messdaten über eine definierte Länge, gebildet werden. Darauf basierend kann gemäß einem weiteren Schritt ein Festsetzen und Einfrieren, insbesondere abspeichern, des gebildeten Mittelwerts, vorzugsweise Referenzwerts, erfolgen. Anschließend kann gemäß einem weiteren Schritt ein Erfassen von Remissionswerten und Bilden weiterer Mittelwerte daraus über eine definierte Länge, beispielsweise 100 Meter, im Zuge des weiteren Spinnvorgangs vorgesehen werden. Dies ermöglicht gemäß einem weiteren Schritt ein Vergleichen der weiteren Mittelwerte mit dem Referenzwert. Dadurch ist es möglich, dass gemäß einem weiteren Schritt ein Bewerten des Vergleichs erfolgt. Bei Überschreiten der Abweichung können Maßnahmen wie das Abstellen der Spinnstelle eingeleitet werden. Insbesondere ist es dabei vorgesehen, dass der Sensor und die damit verbundenen Auswertungen ausschließlich auf Helligkeitssignalen in Verbindung mit dem ermittelten Durchmesser des Garns und/oder Abschattungssignalen beruht.

### Bezugszeichenliste

| | | | |
|---|---|---|---|
| 100 | Verfahren | 307 | Absaugung |
| 101 | Initialisieren | 308 | Spinndüse |
| 102 | Empfangen | 309a | künstliche Lichtquelle |
| 103 | Auswerten | 309b | Leuchtdiode |
| 104 | Erkennen | 309c | Infrarot-Leuchtdiode |
| 105 | Generieren | 310a | erster optischer Sensor |
| 106 | Beenden | 310b | zweiter optischer Sensor |
| | | 310c | dritter optischer Sensor |
| 201 | Helligkeitswertverlauf | 311a | erster Helligkeitssensor |
| 202 | Helligkeitswert | 311b | zweiter Helligkeitssensor |
| 203 | Helligkeitsreferenzwert | 311c | dritter Helligkeitssensor |
| 204 | Abszisse | 312a | erste Foto-Diode |
| 205 | Ordinate | 312b | zweite Foto-Diode |
| | | 312c | dritte Foto-Diode |
| 301 | Spinnmaschine | 313 | Garn |
| 302 | Computerprogramm | 314 | Garnreiniger |
| 303 | computerlesbares Medium | 315a | Kabel |
| 304 | Vorrichtung zur Datenverarbeitung | 315b | weiteres Kabel |
| 305 | Steuergerät | 316 | Messraum |
| 306 | Überwachungsvorrichtung | | |

## Patentansprüche

1. Verfahren (100) zur Erkennung einer Entstehung von wenigstens einer Schwachstelle eines gesponnenen, insbesondere luftgesponnenen, Garns (313) bei einer Spinnmaschine (301), aufweisend die nachfolgenden Schritte:
- Empfangen (102) von mindestens einem Erfassungswert, welcher aus einer optischen Erfassung des Garns (313) resultiert, wobei der mindestens eine Erfassungswert für eine Helligkeit des Garns (313) spezifisch ist,
- Auswerten (103) des mindestens einen Erfassungswerts, um eine Unregelmäßigkeit in Bezug auf die Helligkeit des Garns (313) festzustellen,
- Erkennen (104) der Entstehung der wenigstens einen Schwachstelle auf Basis der festgestellten Unregelmäßigkeit.

2. Verfahren (100) nach Anspruch 1, **dadurch gekennzeichnet, dass** das Auswerten (103) des mindestens einen Erfassungswerts den folgenden Schritt umfasst:
- Vergleichen des mindestens einen Erfassungswerts, der insbesondere einem Helligkeitswert (202) des Garns (313) entspricht, mit mindestens einem Referenzwert, vorzugsweise Helligkeitsreferenzwert (203), um die Unregelmäßigkeit auf Basis einer Abweichung des mindestens einen Erfassungswerts zum mindestens einen Referenzwert festzustellen,
wobei vorzugsweise die Entstehung der wenigstens einen Schwachstelle auf Basis einer quantitativen Auswertung der Abweichung erkannt wird, wobei bevorzugt hierdurch die wenigstens eine Schwachstelle in der Form eines strukturellen Defizits des Garns erkannt wird.

3. Verfahren (100) nach Anspruch 2, **dadurch gekennzeichnet, dass** die Unregelmäßigkeit in Bezug auf die Helligkeit des Garns (313) festgestellt wird, wenn der mindestens eine Helligkeitswert (202) des Garns (313) oder ein davon abgeleiteter Wert den mindestens einen Helligkeitsreferenzwert (203) unterschreitet.

4. Verfahren (100) nach einem der vorhergehenden Ansprüche, **dadurch gekennzeichnet, dass** der mindestens eine Erfassungswert, insbesondere Helligkeitswert (202), wenigstens einen der folgenden Werte umfasst:
- mehrere Helligkeitswerte (202),
- einen Helligkeitswertverlauf (201),
- eine Helligkeitswertänderung,
- eine Helligkeitswertänderungsrate,
und dass vorzugsweise der mindestens eine Referenzwert, insbesondere Helligkeitsreferenzwert (203), wenigstens einen der folgenden Referenzwerte umfasst:
- mehrere Helligkeitsreferenzwerte (203),
- einen Helligkeitsreferenzwertverlauf,
- eine Helligkeitsreferenzwertänderung,
- eine Helligkeitsreferenzwertänderungsrate.

5. Verfahren (100) nach einem der vorhergehenden Ansprüche, **dadurch gekennzeichnet, dass** der mindestens eine Erfassungswert, insbesondere Helligkeitswert (202), einem gemittelten Wert und vorzugsweise der mindestens eine Referenzwert, insbesondere Helligkeitsreferenzwert (203), einem gemittelten Wert entspricht.

6. Verfahren (100) nach einem der vorhergehenden Ansprüche, **dadurch gekennzeichnet, dass** eine Verunreinigung, insbesondere umfassend Fremdpartikel und/oder Fremdfasern, des Garns beim Auswerten (103) berücksichtigt wird, um einen Einfluss der Verunreinigung auf den mindestens einen Erfassungswert zu kompensieren, wobei hierzu vorzugsweise eine Verunreinigungskalibrierung stattfindet, wenn das Spinnen des Garns (313) gestoppt wird.

7. Verfahren (100) nach einem der vorhergehenden Ansprüche, **dadurch gekennzeichnet, dass** das Erkennen (104) der Entstehung der wenigstens einen Schwachstelle zeitlich vor einer vollständigen Ausbildung der wenigstens einen Schwachstelle erfolgt, wobei das Erkennen (104) der Entstehung der wenigstens einen Schwachstelle vorzugsweise in der Form einer Erkennung von fehlenden oder fehlerhaft liegenden Umwindungsfasern des Garns (313) erfolgt.

8. Verfahren (100) nach einem der vorhergehenden Ansprüche, **dadurch gekennzeichnet, dass** das Verfahren ferner folgenden Schritt umfasst:
- Initiieren einer Reaktion auf das Erkennen (104) der Entstehung der wenigstens einen Schwachstelle, wobei hierzu eine Ausgabe generiert wird, die eine, insbesondere pneumatische, Reinigung einer Spinndüse (308) der Spinnmaschine (301) und/oder ein Beenden (106) oder Unterbrechen eines Spinnvorgangs der Spinnmaschine (301), insbesondere nach mindestens einer vorangegangenen Reinigung einer Spinndüse (308) oder der Spinndüse (308) der Spinnmaschine (301), und/oder eine Wartung der Spinnmaschine (301), insbesondere nach mindestens einer vorangegangenen Reinigung einer Spinndüse (308) oder der Spinndüse (308) der Spinnmaschine (301), veranlasst, wenn die Entstehung wenigstens einer weiteren Schwachstelle des Garns (313) erkannt wird.

9. Verfahren (100) nach einem der Ansprüche 2 bis 8, **dadurch gekennzeichnet, dass** das Verfahren (100) ferner die folgenden Schritte umfasst:
- Definieren eines Zeitpunkts eines Spinnvorgangs der Spinnmaschine (301), ab welchem ein mindestens erster Erfassungswert, insbesondere in der Form eines Remissionswerts des Garns (313), empfangen werden soll,
- Definieren einer Länge des Garns (313), für welche der mindestens erste Erfassungswert empfangen werden soll,
- Initiieren des Empfangs (102) des mindestens ersten Erfassungswerts ab dem definierten Zeitpunkt des Spinnvorgangs und für die definierte Länge des Garns (313), um aus dem empfangenen, mindestens ersten Erfassungswert den mindestens einen Referenzwert zu bilden, vorzugsweise durch eine Mittelwertbildung,
- Initiieren des Empfangs (102) von mindestens einem weiteren Erfassungswert für den weiteren Spinnvorgang, insbesondere wiederholt für die oder eine weitere definierte Länge des Garns (313), um aus dem mindestens einem weiteren Erfassungswert zumindest einen aktuellen Erfassungswert zu erhalten, vorzugsweise durch eine Mittelwertbildung,
wobei das Erkennen (104) der Entstehung der wenigstens einen Schwachstelle des Garns (313) auf Basis eines Vergleichs des aktuellen Erfassungswerts mit dem mindestens einen Referenzwert durchgeführt wird, und die Entstehung der wenigstens einen Schwachstelle des Garns (313) vorzugsweise dann erkannt wird, wenn der aktuelle Erfassungswert von dem Referenzwert mit einem definierten Betrag abweicht.

10. Vorrichtung zur Datenverarbeitung (304), die Mittel zur Ausführung des Verfahrens (100) nach einem der Ansprüche 1 bis 9 umfasst.

11. Überwachungsvorrichtung (306), umfassend die Vorrichtung zur Datenverarbeitung (304) gemäß Anspruch 10, wobei es sich bei der Überwachungsvorrichtung (306) insbesondere um einen Garnreiniger (314) handelt.

12. Überwachungsvorrichtung (306) gemäß Anspruch 11, **dadurch gekennzeichnet, dass** die Überwachungsvorrichtung (306) eine künstliche Lichtquelle (309a), insbesondere eine Infrarot-Leuchtdiode (309c), zur Bestrahlung des Garns (313) mit Licht, insbesondere Infrarot-Licht, umfasst, und
dass die Überwachungsvorrichtung (306) zumindest oder genau eine oder zwei optische Sensoren (310a, 310b), vorzugsweise Foto-Dioden (312a, 312b), insbesondere Remissions-Dioden, zur Erfassung des durch das Garn (313) reflektierten Lichts der Lichtquelle umfasst, die benachbart zur Lichtquelle (309a) angeordnet sind, und dass die Überwachungsvorrichtung (306) vorzugsweise einen dritten optischen Sensor (310c), vorzugsweise eine dritte Foto-Diode (312c), umfasst, die von der Lichtquelle (309a) aus gesehen hinter dem Garn (313) angeordnet ist und zur Erfassung einer Abschattung, die sich durch die Anordnung des Garns (313) im Verhältnis zur Lichtquelle (309a) sowie dem Licht der Lichtquelle (309a) ergibt, umfasst.

13. Spinnmaschine (301), insbesondere Luftspinnmaschine, aufweisend mindestens eine Überwachungsvorrichtung (306) gemäß Anspruch 11 oder 12.

14. Computerprogramm (302), umfassend Befehle, die bei der Ausführung des Computerprogramms (302) durch die Vorrichtung zur Datenverarbeitung (304), einen Computer, diesen veranlassen, das Verfahren (100) nach einem der Ansprüche 1 bis 9 auszuführen.

15. Computerlesbares Medium (303), auf dem das Computerprogramm (302) nach Anspruch 14 abgespeichert ist.
